# EUROPÄISCHE PATENTSCHRIFT

(11) **EP 2 802 287 B1**
(45) Veröffentlichungstag und Bekanntmachung des Hinweises auf die Patenterteilung: **26.06.2019**
(21) Anmeldenummer: 12810270.4
(22) Anmeldetag: 20.12.2012
(51) Int. Cl.: A61F 2/95, A61F 2/958, A61F 2/00

(54) **ANORDNUNG UND VERFAHREN ZUR BEREITSTELLUNG EINES STENTS ZUR IMPLANTATION MIT UMHÜLLUNG**
ARANGEMENT AND METHOD FOR PREPARING A STENT FOR IMPLANTATION USING A COVER
AGENCEMENT ET PROCÉDÉ POUR PRÉPARER UN STENT À IMPLANTER, AU MOYEN D'UNE ENVELOPPE

(30) Priorität: 11.01.2012 CH 48122012
(43) Veröffentlichungstag der Anmeldung: 19.11.2014
(73) Patentinhaber: Qvanteq AG, 8005 Zürich (CH)
(72) Erfinder: ZUCKER, Arik, CH-8003 Zürich (CH); BUZZI, Stefano, CH-8049 Zürich (CH); MÄDER, Armin, W., CH-8805 Richterswil (CH); SCHNEEBERGER, Roman, CH-4805 Brittnau (CH); MILLERET, Vincent, CH-8057 Zürich (CH)
(74) Vertreter: Latscha Schöllhorn Partner AG
(86) Internationale Anmeldenummer: PCT/EP2012/076406
(87) Internationale Veröffentlichungsnummer: WO 2013/104509

(56) Entgegenhaltungen:
- WO-A2-01/21103
- US-A1- 2005 278 012
- US-A1- 2006 093 513
- US-A1- 2006 230 592
- US-A1- 2007 014 686
- US-A1- 2007 061 008
- US-A1- 2009 143 857

## Beschreibung

Die vorliegende Erfindung betrifft die Bereitstellung eines Implantats, insbesondere eines Stents, zur Implantation und/oder zur Lagerung eines Implantats vor einer Implantation, eine Anordnung zur Bearbeitung und/oder Bereitstellung eines Implantats sowie eine Verpackung für ein Implantat.

Kleine Implantate, wie Stents, sind in einer Vielzahl medizinischer Anwendungen weit verbreitet. Stents werden beispielsweise zur Behandlung von Läsionen in Blutgefässen oder von Aneurysmen verwendet oder zur Gefässdilatation im Gebiet der perkutanen transluminalen Angioplastie, darunter auch kardiovaskuläre Intervention.

Im Wesentlichen wird zwischen ballon-expandierenden oder selbst-expandierenden Stents unterschieden. Ballon-expandierende Stents werden vor ihrer Implantation auf einen nicht-expandierten Ballon aufgebracht. Hierfür wird beispielsweise der Stent über dem nicht-expandierten Ballon auf einen kleineren Durchmesser komprimiert und gemeinsam mit dem Ballon mittels einer Transporteinrichtung in den Körper eingeführt. Am Behandlungsort, z. B. bei einer Läsion oder einer Gefässklappe, wird der Ballon expandiert, so dass er den Stent aufweitet. Anschliessend kann der Ballon aus dem Körper entfernt werden. Selbst-expandierende Stents bestehen beispielsweise aus einem Metall mit Memory-Effekt. Sie können zum Einführen in ein Körperlumen entgegen ihrer Spannkraft komprimiert und in einen Schlauchkatheter eingesetzt werden. Am Behandlungsort werden sie aus dem Katheter freigesetzt (z.B. aus dem Schlauchkatheter herausgestossen) und springen zurück in ihren expandierten Zustand.

Ferner wird zwischen blanken Metallstents und Stents mit Beschichtungen unterschieden. Soehnlein et al. (Neutrophil-Derived Cathelicidin Protects from Neointimal Hyperplasia; Sci Transl Med 3, 2011) zeigt z. B. einen Stent, der eine Proteinbeschichtung mit dem Protein Cathelicidin (LL37) aufweist. Das Einwachsverhalten des beschichteten Stents wurde in Versuchen an Tieren getestet. Die Versuche zeigten eine geringe Restenose und es wird vermutet, dass Cathelicidin die Reendothelialisation fördert und reguliert und dadurch vor einer neointimalen Hyperplasie nach der Stentimplantation schützt. Die Verwendung von Beschichtungen auf Stents weist jedoch im allgemeinen ihrerseits diverse Nachteile auf, z. B. kann die Oberflächenreibung der Stents erhöht werden, die Beschichtung kann bei der Expansion des Stents Schaden nehmen (reissen, abplatzen, etc.) oder negative Nebenreaktionen mit dem Blutkreislauf erzeugen.

Allen Stents gemeinsam ist es, dass sie zum Einführen in ein Körperlumen einen geringeren Durchmesser aufweisen müssen, als bei der Ausübung ihrer Funktion im Köper. In der Regel werden die Stents vom Hersteller auf einen Katheter vormontiert und verpackt. Es ist aber auch möglich, einen Stent erst kurz vor dem Einsetzen der Stents in das Körperlumen zu komprimieren, d. h. zu crimpen. Bei herkömmlichen Verfahren zur Bereitstellung eines Stents zur Implantation wird der Stent meist in expandiertem oder semi-expandiertem Zustand auf dem Katheter auf einen kleineren Durchmesser komprimiert, der zum Einführen in den Körper eines Patienten geeignet ist. Dieser Vorgang findet üblicherweise unter so genannten Reinraumbedingungen statt. Ein Reinraum ist ein Raum, in dem die Konzentration luftgetragener Partikel, die in den Raum eingebracht werden oder dort entstehen, so gering wie nötig gehalten wird. Die Partikelkonzentration in den Reinräumen wird ständig überwacht. Reinräume werden in verschiedene Klassen eingeteilt, so genannte ISO-Klassen, so darf z.B. in einem Reinraum der Klasse ISO-7 die Anzahl Partikel, welche 0.5 Mikrometer gross sind, nicht mehr als 352'000 pro Kubikmeter betragen. Für 1.0 Mikrometer ist die Obergrenze 83'200 und für 5.0 Mikrometer 2'930 Partikel pro Kubikmeter (nach DIN EN ISO 14644). Kleinere Teilchen als 0.5 Mikrometer werden in dieser Klasse nicht berücksichtigt. Für medizinische Implantate, wie z.B. Stents, wird oft ein solcher ISO-7 Reinraum verwendet.

Ein Reinraum zur Herstellung und Bereitstellung von Stents auf Katheter ist also nicht 100% partikelfrei. Zudem werden gerade für Stents und Katheter viele Produktionsschritte manuell durchgeführt. Da in der Regel der Mensch die grösste Quelle für Partikel und andere Verschmutzungen ist, helfen eine angepasste Arbeitskleidung, die spezifizierte Reinraumklasse einzuhalten.

Die in Blutgefässen implantierten metallischen Stents bergen gewisse Risiken für den Patienten. Unter anderem können Entzündungsreaktionen und/oder Thrombosebildung an den Strukturen des Stents zu einer erneuten Stenose in den Blutgefässen führen. Derartige Komplikationen werden u. a. durch Verunreinigungen des Stents, der Einführvorrichtung oder anderer Elemente, die mit dem Stent in Berührung kommen, beim Einsetzen des Stents in ein Körperlumen verursacht. Der Stent und die Transporteinrichtung sollen daher frei von jeglicher Kontamination sein, d. h. beispielsweise keinen Staub, keine Fasern oder Partikel im Allgemeinen aufweisen. Dazu ist beispielsweise in der US 2007/0014686 A1 ein System zum Sterilisieren bzw. Dekontaminieren von Objekten wie unter anderem Stents beschrieben.
Derartige Verunreinigungen können bei herkömmlichen Verfahren zur Bereitstellung eines Stents zur Implantation z. B. auch nach einer Oberflächenbehandlung und Reinigung des Stents, beim Transfer des Stents, beim Crimpen oder Einsetzen auf oder in einen Katheter entstehen. Beispielsweise können "natürliche", durch die im Reinraum verrichtete Arbeit entstehende Kontaminationen, z. B. von Arbeits-Handschuhen oder diverse Partikel aus der Atmosphäre, am Stent haften bleiben.
Durch optische Kontrollen nach der Stentmontage auf dem Katheter können allfällige Partikel erkannt und allenfalls mit einem sauberen Gas (z.B. öl- und partikelfreie, ionisierte Luft) aus einer Düse strömend entfernt werden.
Partikel, welche sich jedoch zwischen dem montierten Stent und dem Katheter befinden, können unter Umständen nicht erkannt werden. So können sich u.a. Partikel bei einem ballonexpandierenden Stent zwischen montiertem Stent und Ballon und bei einem selbstexpandierenden Stent zwischen montiertem Stent und Innenschlauch des Schlauchkatheters befinden. Kleinstpartikel, welche nicht unbedingt optisch erkannt werden können und auch nicht unter die Bedingung der im entsprechenden Reinraum angewendeten ISO Norm fallen, verbleiben ebenfalls auf den Oberflächen von Stent und Katheter. Zudem werden diese optischen Kontrollen ebenfalls oft manuell durchgeführt, was eine zusätzliche Fehlerquelle bedeuten kann.
Aus dem Stand der Technik sind verschiedene Methoden bekannt, gewisse Arten der Verunreinigung an einem Implantat oder einer Transporteinrichtung für ein Implantat zu vermeiden. In der WO 2006/086709 ist beispielsweise eine Vorrichtung zum Komprimieren von Nitinolstents gezeigt. Der expandierte Stent wird von einer ersten Temperatur auf eine zweite Temperatur herab gekühlt und anschliessend komprimiert. Dabei besteht das Problem, dass beim Kühlen gasförmige Stoffe, wie z.B. Wasserdampf oder andere sich in der Luft befindende Sublimate auf der Oberfläche des Stents kondensieren und sogar gefrieren können und somit das Komprimieren behindern und/oder die Stentoberfläche beschädigen, was u.a. bei Beschichtungen o.ä. der Fall sein kann. Um dies zu verhindern werden der Stent und die Crimpvorrichtung in einer abgeschlossenen Kammer vorgesehen und jegliche gasförmigen Stoffe, welche beim Kühlvorgang
kondensieren können aus der Kammer soweit möglich entfernt, wie etwa Wasserdampf und Kohlendioxid. Erst anschliessend wird der Stent auf die Kompressionstemperatur gekühlt. Zum Entfernen der gasförmigen Stoffe wird die Kammer mit einem Gas versorgt, wie etwa Stickstoff, so dass sich der Stent und die Crimpvorrichtung in einer Stickstoffumgebung befinden. Dies ist jedoch nicht ausreichend, denn es können immer noch kleine Kontaminationspartikel im Mikrometerbereich präsent sein, welche z.B. schon vor dem Gasaustausch auf der Stentoberfläche oder auf der Ballonoberfläche liegen, jedoch den Crimpvorgang nicht beeinflussen.

In der US 2006/0183383 ist eine Crimpvorrichtung zum Komprimieren eines Stents gezeigt, die eine Schutzschicht zwischen Stent und Crimpelementen der Vorrichtung verwendet, um den Stent beim Komprimieren zu schützen. Die Schutzschicht wird in Form eines rollbaren Materials in das Innere der Crimpvorrichtung eingebracht und kommt zwischen dem Stent und den Crimpelemente zu liegen. Die Schutzschicht ist an Ihren Enden jeweils auf einer Rolle aufgerollt und wird mit Ihrem zwischen den Rollen gespannten Bereich durch eine Längsöffnung, die z. B. durch Herausnehmen eines Crimpelements entsteht, durch einen Druckstrahl eines Gases zwischen den Elementen hindurch in das Innere der Crimpvorrichtung hinein geblasen. Sobald die Crimpvorrichtung zum Komprimieren des Stents geschlossen wird, muss der Gasstrom eingestellt werden und das Gas kann durch die vordere und hintere Öffnung der Crimpvorrichtung entweichen, so dass der Stent der Atmosphäre ausgesetzt ist. Nach dem Komprimieren wird der Stent aus der Crimpvorrichtung entnommen und kann beim Transfer wiederum einer Kontamination unterliegen.

Ferner ist in der US 2003/0187493 eine Verpackungshülle für einen komprimierten Stent und einen Katheter, auf dem der Stent montiert ist, gezeigt. Die Hülle berührt die Oberfläche des Stents, bzw. eine Beschichtung auf der Oberfläche des Stents, nicht, um diese nicht zu beschädigen (v.a. wenn es sich z.B. um eine Beschichtung handelt) oder zu verunreinigen, beispielsweise wenn der Stent in oder auf eine Transporteinrichtung zur Implantation ein- oder aufgebracht wird. Verunreinigungen in Form von Mikropartikel (oder kleiner), die auf dem Stent bereits vor dem Verpacken vorhanden waren, verbleiben somit auf dem Stent.

Weitere Vorrichtungen zum Komprimieren eines Stents oder zu dessen Aufbewahrung sind z. B. aus US 6,968,607, US 2008/0072653, WO 2004/066876 oder EP 0910425 bekannt.

Bei den bekannten Vorrichtungen und Verfahren zur Bearbeitung und Bereitstellung kleiner Implantate, wie Stents, wird dieses zwar innerhalb der Vorrichtung oder während des Verfahrens in einer reinen Umgebung gehalten und Verunreinigungen werden weitgehend vermieden. Nach der Bearbeitung, z. B. beim Transfer von einer Arbeitseinheit zu einer nächsten oder bei einer einfachen Sichtkontrolle eines Implantats können sich jedoch erneut Verunreinigungen auf der Oberfläche des Implantats ablagern. Im Falle eines Stents können derartige Verunreinigung beispielsweise bei einem Crimpprozess zwischen dem Stent und seinem Transportkatheter zu liegen kommen und werden mit dem Stent in ein Körperlumen eingeführt, wo sie zu Komplikationen führen können, wie vorher erläutert wurde.

Es ist eine Aufgabe der vorliegenden Erfindung, eine Anordnung und ein Verfahren zur Bereitstellung eines Implantats zur Implantation zu schaffen, welche eine Verunreinigung von Implantaten bei der Vorbereitung zur Implantation oder bei der Verpackung reduzieren, nach einem Reinigungsprozess erneute Verunreinigungen verhindern, die Qualität einer Oberfläche eines Implantats bewahren und die einfach in der Handhabung und Ausführung sind. Weiter ist es eine Aufgabe der Erfindung eine Verpackung für ein Implantat zu schaffen, die eine rekontaminationsfreie Lagerung ermöglicht, den Bearbeitungsprozess unterstützt und unkompliziert in ihrer Anwendung ist.

Diese Aufgabe wird von der Erfindung durch eine Anordnung und ein Verfahren zur Bereitstellung eines Implantats zur Implantation nach den Ansprüchen 1 und 11 gelöst. Vorteilhafte Ausgestaltungen und verschiedene Ausführungsbeispiele sind in den abhängigen Ansprüchen beschrieben.

Die Anordnung und das Verfahren sind grundsätzlich für Implantate im allgemeinen geeignet, insbesondere aber für kleine Implantate, wie etwa Gefässprothesen. Erfindungsgemäss beziehen sich die Anordnung und das Verfahren auf Stents. Stents weisen im Wesentlichen ein proximales Ende und ein distales Ende auf, wobei sich zwischen den Enden ein Stentlumen mit komprimierbarem Durchmesser erstreckt, das sich in der Regel aus einzelnen Stegen aufbaut. Wie eingangs beschrieben, werden Stents auf einer Transporteinrichtung, z. B. einem Katheter, angeordnet, um innerhalb eines Körperlumens transportiert werden zu können. Die vorliegende Erfindung ist insbesondere geeignet, um Verunreinigungen zwischen Stentoberfläche und Katheteroberfläche, z. B. in Form von Mikropartikeln wie vorher beschrieben, zu vermeiden.

Bei einem Verfahren zur Bereitstellung eines Implantats, insbesondere eines Stents, zur Implantation in ein Körperlumen, wird das Implantat innerhalb einer Umhüllung einer Bearbeitungseinheit oder einer Verpackung zugeführt und vorzugsweise auch wieder entnommen. Dabei wird das Implantat zwischen einer vorderen und einer hinteren Öffnung der Umhüllung gelagert und ein Strom eines definierten Mediums wird durch die Umhüllung geleitet. Der Strom umströmt das Implantat in der Umhüllung und die Bearbeitungseinheit wirkt von ausserhalb der Umhüllung auf das Implantat ein.

Dabei wird erfindungsgemäss eine Anordnung zur Bereitstellung eines Stents zur Implantation in ein Körperlumen verwendet, die wenigstens eine Bearbeitungseinheit zur Bearbeitung des Stents, eine Umhüllung mit einer vorderen Öffnung und einer hinteren Öffnung, und wenigstens eine Strömungseinheit zur Erzeugung eines Stroms eines definierten Mediums durch die Umhüllung umfasst, wobei der Strom den Stent innerhalb der Umhüllung vorzugsweise von allen Seiten umströmt. Die Strömungseinheit kann unmittelbar an die Umhüllung angeschlossen sein. Alternativ kann zwischen der Strömungseinheit und der Umhüllung z. B. eine Verpackung angeordnet sein, so dass ein Stent aus der Umhüllung in die Verpackung transferiert werden kann, ohne dass eine Unterbrechung des Stroms um den Stent erfolgen muss. Die Umhüllung ist zumindest teilweise an oder in der Bearbeitungseinheit angeordnet oder kann an oder in dieser angeordnet werden, wobei das Implantat innerhalb der Umhüllung zwischen deren Öffnungen gelagert ist. Die Strömungseinheit zur Erzeugung des Stroms durch die Umhüllung ist zumindest an eine Öffnung der Umhüllung angeschlossen. Die Öffnungen der Umhüllung bilden dabei eine Einström- und eine Ausströmöffnung. Die Umhüllung kann ein herkömmliches Anschlusssystem zum Anschliessen der Strömungseinheit aufweisen, wie etwa eine Steck- oder Schraubverbindung. Ein Stent wird vorzugsweise von dem Strom gleichzeitig von allen Seiten umströmt, also auf seiner Aussen- und Innenumfangsfläche und auch zwischen einzelnen Stegen des Stents. Nachfolgend wird die Erfindung beispielhaft für ein Implantat in Form eines Stents beschrieben. Die Anordnung kann jedoch auch vorteilhaft für andere Implantate, wie z. B. Grafts oder Zahnimplantate, verwendet werden.

Experimentell konnte nachgewiesen werden, dass gemäss der Erfindung zur Implantation bereitgestellte Stents eine reduzierte Restenose zeigen. Dies wird darauf zurückgeführt, dass die nach der Erfindung vorbereiteten und gelagerten Stents im Vergleich zu herkömmlich bereitgestellten und gelagerten Stents einen höheren Grad an Sauberkeit zum Zeitpunkt der Implantation aufweisen, d. h. die Kontamination deutlich reduziert ist. Mit Hilfe der Erfindung kann ein einheitlicher Reinheitsgrad über die gesamte Stentoberfläche beibehalten oder erreicht werden. Es bleiben keine Partikel als Keimzellen für ein unregelmässiges Einwachsen zurück. Insbesondere bei hydrophylisierten Stents ist es mit dem Vorgehen und der Anordnung nach der Erfindung möglich, den hohen Reinheitsgrad der hydrophylisierten Oberfläche bis zum Einführen des Stents in das Körperlumen beizubehalten.

Erfindungsgemäss ist gleichzeitig mit dem Stent auch ein Katheter innerhalb der Umhüllung zwischen deren Öffnungen angeordnet und wird von dem definierten Medium umströmt, wie nachfolgend genauer ausgeführt wird.

Bei dem Verfahren und der Anordnung nach der vorliegenden Erfindung kann der Stent nach der Reinigung, bei der Bearbeitung, der Lagerung und bis zum Einführen in ein Körperlumen einem Strom eines definierten Mediums unterliegen, wobei er vorzugsweise bei jedem Handhabungsschritt innerhalb der Umhüllung verbleibt. So kann beispielsweise auch während dem Einbringen in eine Verarbeitungseinheit und während der Verarbeitung ein Strom herrschen, der Kontaminationspartikel aus der Umhüllung abtransportiert. Die Ablagerung von Verunreinigungen wird somit unterbunden.

Der Stent kann z. B. innerhalb der Umhüllung angeordnet und in dieser von einer Reinigungseinheit gereinigt werden. Zur Reinigung wird beispielsweise eine Ultraschalleinheit verwendet, die von ausserhalb der Umhüllung in bekannter Weise auf den Stent einwirkt. Gleichzeitig kann der Stent innerhalb der Umhüllung mit einer Lösung umspült werden. Es kann eine basische Lösung, z. B. mit 3% Deconex in destilliertem Wasser gelöst, verwendet werden. Es ist auch möglich, eine saure Lösung zu verwenden, wie z. B. konzentrierte Schwefelsäure (z. B. 96%). Das Material der Umhüllung wird auf die Art der Lösung abgestimmt. Im Falle von Schwefelsäure und/oder Deconex Lösung kann z. B. ein Teflon-Schlauch (PTFE) verwendet werden. Die Lösung kann somit ein definiertes Medium im Sinne der Erfindung darstellen. Grundsätzlich
sind auch andere herkömmliche Reinigungsverfahren anwendbar. Im Allgemeinen kann die Umhüllung aus Kunststoff, etwa PA und PE, bestehen. Gute Ergebnisse wurden mit PTFE und PFA erzielt.

Der Stent verbleibt somit bei und nach der Reinigung innerhalb der Umhüllung und der anliegende Strom des definierten Mediums umspült den Stent. In dieser geschützten Atmosphäre kann der Stent in eine Bearbeitungseinheit eingebracht werden, ohne zwischenzeitlich einer kontaminierenden Atmosphäre ausgesetzt zu sein.

Dabei kann das definierte Medium auch während der einzelnen Schritte des Bereitstellungsverfahrens für den Stent gewechselt werden. Beispielsweise kann vor und/oder nach einem Strom bestehend aus einer Lösung zur Reinigung ein anderer, z. B. Stickstoffstrom, durch die Umhüllung geleitet werden, so dass der Stent ohne Unterbrechung von unterschiedlichen, nacheinander folgenden Strömen umspült wird. Die vordere Öffnung der Umhüllung kann hierfür z. B. mit mehreren Zugängen, ebenfalls Öffnungen, versehen sein, durch die verschiedene Medien in die Umhüllung eingeleitet werden können. Es kann z. B. ein Y-Element mit zwei Zugängen an die vordere Öffnung der Umhüllung angeschlossen werden und an jeden Zugang eine Strömungseinheit für ein bestimmtes Medium angeschlossen werden. Grundsätzlich können auch drei oder mehr Zugänge für drei oder mehr Strömungseinheiten vorgesehen werden. Die verschiedenen Medien können auch innerhalb der Umhüllung vermischt werden. Die Mischverhältnisse können dabei z. B. durch Regelung der Strömungseinheiten bestimmt werden.

In einer Ausführungsform ist die Umhüllung schlauchartig geformt sowie flexibel und biegsam ausgebildet. Die Umhüllung kann sich an beiden Enden des Implantats, bzw. des Stents, weit über diesen hinaus erstrecken, so dass sich die Umhüllung über mehrere Bearbeitungseinheiten erstrecken kann. Durch die Flexibilität der Umhüllung kann von ausserhalb der Umhüllung auf den Stent eingewirkt werden, wobei die Umhüllung nachgibt und der Stent z. B. komprimiert werden kann. Die Oberfläche des Stents bleibt jedoch geschützt. Es können Haltemittel zur Positionierung des Stents innerhalb der Umhüllung oder zum Ergreifen des Stents von aussen vorgesehen sein. Diese können z. B. in Form von ein oder mehrerer Verjüngungen des Durchmessers der Umhüllung vorgesehen sein, beispielsweise durch Zusammendrücken der Umhüllung von aussen etwa durch Klemmen oder manuell. Der Stent kann z. B. an den Verjüngungen lose gelagert werden. Somit kann der Stent innerhalb der Umhüllung mit geringem Spiel beweglich gelagert sein, so dass er für den Strom des definierten Mediums von allen Seiten zugänglich bleibt. Der Stent kann auch von ausserhalb der Umhüllung an seiner Umfangsfläche lösbar gehalten werden. Im wesentlichen sind die Haltemittel derart ausgestaltet, dass der Stent trotz einer Fixierung durch die Haltemittel möglichst allseits umströmt werden kann.

Gleichzeitig mit dem Stent kann eine Transporteinrichtung zum Einführen des Implantats in ein Körperlumen gemeinsam mit dem Implantat in der Umhüllung zwischen deren Öffnungen vorgesehen sein. Im Fall eines Ballonkatheters als Transporteinrichtung kann folgendermassen vorgegangen werden. Der Stent wird in die Umhüllung eingebracht und vom Medium umströmt. Anschliessend wird der Ballonkatheter eingebracht und ebenfalls umströmt. Nach einer Weile, während Stent und Ballon umströmt wurden und sich somit keine Partikel mehr auf deren Oberfläche befinden, kann der Ballon in das Stentlumen hineingeschoben werden. Der Ballon kann dann etwas geöffnet, d. h. inflatiert werden, so dass der Stent durch Haftreibung auf dem Ballon verbleibt. Stent und Ballon werden dann als Einheit in die Crimpeinheit geschoben und im Durchmesser reduziert.

Als Umhüllung kann z. B. ein Schlauch aus PTFE, PFA, PE oder PA verwendet werden. Der Schlauch wird vor seiner Verwendung als Umhüllung für den Stent gereinigt. Es ist vorteilhaft, wenn die Umhüllung transparent ist, um den Stent in der Umhüllung einer Sichtkontrolle unterziehen zu können. Vorzugsweise weist die Umhüllung eine Wanddicke von 0.01mm bis 0.09mm auf, insbesondere von 0.02mm bis 0.06mm. In diesem Bereich ist eine Umhüllung ausreichend flexibel und biegsam, um die erforderlichen Bearbeitungsschritte nicht zu behindern, aber ausreichend robust, um bei einer Bearbeitung des Stents nicht zu reissen. Besonders geeignet ist z. B. ein PTFE-Schlauch mit einer Wanddicke von 0.04mm.

Erfindungsgemäss umfasst die Bearbeitungseinheit eine Crimpeinheit, welche einen Stent komprimiert, um ihn auf einem Ballon-Katheter oder in einem Katheter für selbstexpandierende Stents unter zu bringen. Die kann Crimpeinheit Elemente aufweisen, die um eine Achse angeordnet sind und zumindest zum Teil relativ zu einander radial zur Achse beweglich sind, wodurch sich der freie Raum, den die Elemente einschliessen, verkleinert. Die Elemente der Crimpeinheit umfassen den in der Umhüllung gelagerten Stent und sind von einer aufgeweiteten Position, in der der Stent ungecrimpt, bzw. unkomprimiert ist, in eine geschlossene Position, in der der Stent im Durchmesser komprimiert ist, radial beweglich. In der aufgeweiteten Position der Elemente weist der freie Raum, den sie umschliessen, mindestens einen so grossen Durchmesser auf, dass der Stent in expandiertem Zustand in diesem Raum untergebracht werden kann. In der geschlossenen Position der Elemente, sind die Elemente soweit radial nach innen bewegt, dass der freie Raum auf einen Durchmesser verringert wird, der dem Durchmesser entspricht, der für einen komprimierten, bzw. gecrimpten, Stent vorgesehen ist.

Die Elemente der Crimpeinheit können als schwenkbare Backen oder als Segmente ausgebildet sein, die ringförmig um eine Achse angeordnet sind und in Richtung der Achse beweglich sind. Crimpeinheiten sind aus dem Stand der Technik in unterschiedlichen Ausführungen bekannt.

Ein Stent innerhalb seiner Umhüllung kann z. B. in radialer oder axialer Richtung in eine Crimpeinheit eingesetzt werden. Vorzugsweise erstreckt sich die Umhüllung durch die Crimpeinheit und tritt an sich gegenüberliegenden Seiten der Crimpeinheit aus dieser aus. Die Umhüllung steht somit aus der Crimpeinheit hervor. So kann die Strömungseinheit an der Umhüllung angeschlossen bleiben und der Stent kann durch Handhabung der Umhüllung innerhalb der Crimpeinheit manipuliert werden, um ihn z. B. in eine gewünschte Lage relativ zur Einheit zu bringen. Dabei kommen die Crimpelemente ausserhalb der Umhüllung über dem Stent zu liegen und können auf diesen einwirken, ohne dass der Strom des definierten Mediums unterbrochen werden muss.

Bei einer Ausführungsform der Anordnung nach der vorliegenden Erfindung sind mehrere Bearbeitungseinheiten in Folge vorgesehen, wobei sich die Umhüllung durch die mehreren Bearbeitungseinheiten erstreckt. Das Implantat, bzw. der Stent, kann somit innerhalb der Umhüllung von einer Bearbeitungseinheit zu einer nächsten bewegt werden. Alternativ kann die Umhüllung durch die Bearbeitungseinheiten bewegt werden, bis ein innerhalb der Umhüllung im Wesentlichen stationärer Stent gemeinsam mit der Umhüllung von einer Bearbeitungseinheit zu einer nachfolgenden Bearbeitungseinheit oder auch in eine Verpackung bewegt wurde. Während diesem Transfer kann der Strom des definierten Mediums kontinuierlich den Stent umströmen, so dass die Ablagerung von Verunreinigungen vermieden wird.

Grundsätzlich ist es aber auch möglich, dass die Umhüllung aus einer Bearbeitungseinheit entnehmbar ist, so dass ein in der Umhüllung befindliches Implantat, bzw. ein Stent, von einer Bearbeitungseinheit zu einer nachfolgenden Bearbeitungseinheit transferiert werden kann, ohne dabei einer Verunreinigung ausgesetzt zu sein.

Als Bearbeitungseinheit können in der Anordnung z. B. eine Crimpeinheit, wie oben beschrieben, eine Reinigungseinheit, eine Einheit zum Temperieren (Erhöhen oder Erniedrigen der Temperatur) des Implantats oder eine Verpackungseinheit, bzw. eine Verpackung, vorgesehen sein. Je nach Anforderungen an das Implantat können auch andere Bearbeitungseinheiten vorgesehen sein.

Die Strömungseinheit zur Erzeugung eines Stroms eines definierten Mediums durch die Umhüllung kann in einer einfachen Ausführung durch einen Behälter vorgesehen sein, in welchem sich das Medium befindet und mittels erzeugtem Druck/Überdruck oder Gravitation durch eine Öffnung in die Umhüllung einströmt. Als definiertes Medium kann ein Gas, z. B. Stickstoff, oder eine Flüssigkeit verwendet werden. Die Zusammensetzung des Mediums kann auf die Anforderungen des Implantats abgestimmt werden. Vorzugsweise wird ein inertes Medium verwendet, das gegenüber der Oberfläche eines Stents innerhalb der Umhüllung nicht reaktiv ist. Hierfür kann z. B. sterile Kochsalzlösung in physiologischer Konzentration von 0.9% NaCl verwendet werden. Diese hat den Vorteil, dass der Stent in einer physiologisch geeigneten Umgebung gehalten wird. Es kann auch WFI-Wasser (water for injection) verwendet werden. Ferner können dem Medium definierte Substanzen beigemischt werden, beispielsweise durch eine Beimischung innerhalb der Strömungseinheit oder innerhalb der Umhüllung, wie vorher am Beispiel einer Reinigung beschrieben. Durch die definierten Substanzen kann z. B. die Oberfläche eines Stents konserviert werden. Es können aber auch Substanzen verwendet werden, um die Oberflächeneigenschaften eines Stents zu beeinflussen, beispielsweise kann Oxidationsmittel (z.B. Wasserstoffperoxid, also H₂O₂, oder aber auch Salpetersäure, etc.) verwendet werden, um die Stentoberfläche zu oxidieren. Die Strömungseinheit kann eine Druckregelung zur Regelung des Drucks innerhalb der Umhüllung aufweisen. Somit kann sicher gestellt werden, dass ein gleichbleibender Strom durch die Umhüllung strömt, auch wenn der Durchmesser der Umhüllung während der Bearbeitung verändert wird. Bei einem Verschluss der Umhüllung kann die Druckregelung den Strom auch abstellen. Die Stärke des Stroms kann auch variiert werden, z. B. in Abstimmung auf unterschiedliche Bearbeitungsschritte vor einer Verpackung des Stents.

Nach der Erfindung wird das Implantat vorzugsweise umströmt während das Implantat gereinigt, bearbeitet, einer Verpackung zugeführt und/oder zwischen Einheiten transferiert wird. Vorzugsweise erzeugt die Strömungseinheit innerhalb der Anordnung einen retrograden Strom des Mediums in Bezug auf die Transportrichtung eines Implantats innerhalb der Anordnung. Der Strom verläuft somit z. B. innerhalb einer Anordnung von einer Bearbeitungseinheit in Richtung einer Reinigungseinheit. Die Reinigungseinheit ist demnach in Strömungsrichtung nach einer Bearbeitungseinheit vorgesehen. Vorzugsweise verläuft der Strom auch durch eine Verpackungseinheit, bzw. in oder durch die Verpackung selbst, und von dieser in Richtung zur Reinigungseinheit.

Die Verpackung ist vorzugsweise in Strömungsrichtung zu vorderst angeordnet, also z. B. als erste Einheit nach der Einströmöffnung für das definierte Medium. Die Verpackungseinheit kann hierfür mit einer Öffnung an die Strömungseinheit und mit einer gegenüberliegenden Öffnung an eine Öffnung der Umhüllung angeschlossen sein, so dass sich die Umhüllung an die Verpackungseinheit in Strömungsrichtung anschliesst. Die Verpackungseinheit wird somit von dem definierten Medium umströmt. So wird auch die Verpackung von möglichen Verunreinigungen / Kontaminationspartikel gereinigt und ihre Oberfläche bleibt bis zur Einführung des Implantates, bzw. des Implantats montiert auf einer Transporteinrichtung, sauber. Die Strömungsrichtung verläuft in der Regel in Längsrichtung des Implantats, insbesondere des Stents, und somit in Längsrichtung einer Verarbeitungsachse, wie etwa der Längsachse einer Crimpeinheit. Sofern eine Transporteinrichtung für den Stent innerhalb der Umhüllung vorgesehen ist, ist auch diese in Längsrichtung orientiert. Das Implantat ist somit während der Bereitstellung des Implantats zur Implantation zu jeder Zeit von frischem, unkontaminierten Medium umströmt. Dabei liegt vorzugsweise ein kontinuierlicher Strom innerhalb der Umhüllung an. Die Strömungsgeschwindigkeit kann konstant oder variabel einstellbar sein. So kann auf unterschiedliche Gegebenheiten im Ablauf der Bereitstellung des Stents zur Implantation eingegangen werden. Beispielsweise ist es vorteilhaft, während des Transfers zwischen verschiedenen Bearbeitungseinheiten oder zu einer Verpackung eine erhöhte Strömungsgeschwindigkeit anzulegen, um während dessen jegliche Partikel, die zu einer Kontamination führen könnten mittels des Stroms von dem Stent fern zu halten. Grundsätzlich ist es denkbar, dass die Strömungsrichtung des Stroms in umgekehrter Richtung verläuft oder umgekehrt werden kann. Ferner ist es denkbar unterschiedliche Medien, bzw. Substanzen, abwechselnd und / oder aus unterschiedlichen Richtungen durch die Umhüllung strömen zu lassen. Dies kann beispielsweise durch dieselbe Strömungseinheit erfolgen oder durch weitere Strömungseinheiten ermöglicht werden. Die weiteren Strömungseinheiten können am selben Ende oder am gegenüberliegenden Ende einer Umhüllung vorgesehen sein.

Es ist auch möglich, die Temperatur des Stroms während der Bearbeitung des Stents zu verändern. Die Temperaturänderung kann dabei auch zur Bearbeitung des Stents herangezogen werden. Insbesondere bei der Bearbeitung von selbst-expandierenden Stents kann eine Erniedrigung der Temperatur des Mediums zur Bearbeitung des Stents eingesetzt werden. Der Stent kann dadurch z. B. unter die Austenit-FormationsTemperatur (AF-Temperatur) des Stentmaterials gekühlt werden, um den Memory-Effekt des Materials auszuschalten. Für Nitinol Stents ist diese Temperatur beispielweise auf unterhalb der Raumtemperatur eingestellt. Nach dem Kühlen eines solchen Stents kann in einem weiteren Bearbeitungsschritt ein Schlauchkatheter über dem Stent angeordnet werden, während der Strom den komprimierten Stent umströmt.

Gemeinsam mit dem Implantat kann auch eine Transporteinrichtung, wie etwa ein Einführkatheter oder ein Transportkatheter für Ballonexpandierende oder selbst-expandierende Stents, innerhalb der Umhüllung angeordnet und somit von dem Strom umströmt werden. Die Transporteinrichtung ist somit ebenfalls vor Verunreinigungen geschützt bis diese zum Einführen des Stents in ein Körperlumen verwendet wird.

Nach der Erfindung ist es dabei besonders vorteilhaft, dass der Stent auf oder in der Transporteinrichtung angebracht werden kann, während er von einem Strom definierten Mediums umströmt wird, insbesondere entlang der Längsachse des Stents. Kontaminationspartikel, die sich zwischen Stent und Transporteinrichtung befinden, können somit durch den Strom entfernt und z. B. bei einem Crimpprozess nicht zwischen Transporteinrichtung und Stent eingeklemmt werden, wo sie erst bei der Freigabe des Stents innerhalb eines Körperlumens gelöst werden. Dabei ist es vorteilhaft, dass der Strom während der gesamten Bearbeitung kontinuierlich erfolgen kann. Vorzugsweise wird aber zumindest vor und/oder während der Positionierung des Stents auf oder in der Transporteinrichtung ein Strom erzeugt, der den Stent umströmt und Kontaminationen fern hält.

Nach einem Bearbeitungsprozess kann das Implantat, bzw. das Implantat gemeinsam mit der Transporteinrichtung, einer Verpackungseinheit oder direkt einer Verpackung zugeführt werden. Vorzugsweise wird dabei zumindest ein Teil der Umhüllung innerhalb der Verpackung angeordnet. Das Implantat verbleibt somit auch innerhalb der Verpackung in der schützenden Umhüllung. Die Verpackung wird über der Umhüllung geschlossen, so dass die Öffnungen der Umhüllung verschlossen werden. Bis zum Verschliessen der Verpackung kann der Strom die Umhüllung durchströmen. Überstehende Teile der Umhüllung können nach dem Verschliessen abgelängt werden. Ein derart bearbeiteter und verpackter Stent ist somit weitgehend frei von kontaminierenden Partikeln und kann nach der Entnahme aus der Verpackung direkt einem Körperlumen zugeführt werden, ohne dass weitere Bearbeitungsschritte, wie Reinigung oder Komprimieren nötig sind.

Nach der Erfindung ist demnach eine Verpackung eines Implantats zur Implantation in ein Körperlumen vorgesehen, die eine schlauchförmige flexible Umhüllung mit einer vorderen Öffnung und einer hinteren Öffnung, zwischen welchen das Implantat gelagert ist, aufweist, wobei die Umhüllung innerhalb der Verpackung vorgesehen ist und die Öffnungen durch die Verpackung oder Teile der Verpackung verschlossen sind. Zumindest an einer der Öffnungen der Umhüllung kann ein Anschluss für eine Strömungseinheit zur Erzeugung eines Stroms eines definierten Mediums durch die Umhüllung vorgesehen sein.

Alternativ kann die Verpackung zwei Öffnungen aufweisen, die zwischen sich einen Verpackungsraum einschliessen, wobei die Umhüllung an eine erste Öffnung und die Strömungseinheit an eine zweite Öffnung der Verpackung angeschlossen sein kann. Der Strom des definierten Mediums fliesst somit von der Strömungseinheit durch die Verpackung und weiter von der Verpackung durch die Umhüllung. Wiederum ist der Stent zu jeder Zeit während der Bearbeitung und der Verpackung von dem Strom umspült und durch diesen geschützt, während er von der Umhüllung in die Verpackung transferiert wird. Anschliessend werden die Öffnungen der Verpackung verschlossen, so dass der Stent und auch die zugehörige Transporteinrichtung in der geschützten Umgebung gelagert werden können.

Die Erfindung betrifft ferner ein Implantat in Form eines Stents, der nach dem oben beschriebenen Verfahren zur Implantation bereit gestellt ist. Durch das Bereitstellungsverfahren kann ein hoch reiner Stent zur Verfügung gestellt werden, der ein einheitliches, bzw. periodisch regelmässiges Oberflächenbenetzungsverhalten aufweist. Im Vergleich zu herkömmlich bereitgestellten Stents verbessert eine erfindungsgemäss einheitliche Stentoberfläche das Verhalten des Stents beim Einwachsen nach seiner Implantation und wirkt einer Restenose entgegen. Ein experimenteller Nachweis hierfür konnte durch eine in vivo Studie erbracht werden, bei der erfindungsgemässe Stents in eine Koronararterie bei Schweinen eingesetzt wurden und das Einwachsen beobachtet wurde, wie nachfolgend genauer ausgeführt wird. Als überraschendes Ergebnis konnte gezeigt werden, dass das Einwachsverhalten von Stents wesentlich stärker von der Reinheit eines Stents abhängig ist, als bisher vermutet wurde. Wie die Messergebnisse zeigen, werden überraschend gute Resultate bei Stentbehandlungen mit blanken Metallstents erzielt. Bisher wurde angenommen, dass derartige Ergebnisse nur durch spezielle Präparation der Oberfläche, z. B. durch Beschichtung, etwa eine Medikamenten-Beschichtung, der Stents erreicht werden können. Die Experimente zeigen jedoch, dass der Erfolg einer Behandlung eines Körperlumens mit einem Stent in erster Linie von der Reinheit des Stents bestimmt wird.

In einer vorteilhaften Ausgestaltung weist die Gesamtoberfläche des Stents eine einheitliche Reinheit auf, welche weniger Partikel pro Oberflächeneinheit aufweist, als es einer herkömmlich verwendeten Reinraumklasse (z.B. 7) der ISO (International Standard Organisation) entspricht. Das heisst, mit dem vorgeschlagenen Verfahren kann eine höhere Reinheit der Stentoberfläche erzielt werden, als es in Reinräumen der höchsten üblicherweise verwendeten Reinheitsklassen möglich ist, ohne dass ein solcher Reinraum oder auch Reinraum einer tieferen Stufe erforderlich ist. Insbesondere kann der Stent auch weniger Kohlenstoffmoleküle und Partikel einer Grösse unter 0,05 Mikrometer aufweisen, als es einer Reinraumklasse 1 der ISO (International Standard Organisation) entspricht.

Die Oberflächencharakteristik des Stents kann vorteilhaft durch ein Verfahren und eine Vorrichtung gemäss der parallelen Anmeldung der Anmelderin mit dem Titel "Verfahren und Vorrichtung zur Bestimmung einer Oberflächencharakteristik an Stents und Stent mit definierter Oberflächencharakteristik" (Anmeldenummer CH 00049/12) verifiziert werden. Die Offenbarung dieser Anmeldung in Bezug auf die Oberflächencharakteristik eines erfindungsgemässen Stents und der Bestimmung der Oberflächenbeschaffenheit wird vollumfänglich mit eingeschlossen.

Bevorzugte Ausführungsformen der Erfindung werden im Folgenden anhand der Zeichnungen dargestellt, die lediglich zur Erläuterung dienen und nicht einschränkend auszulegen sind. Aus den Zeichnungen offenbar werdende Merkmale der Erfindung sollen einzeln und in jeder Kombination als zur Offenbarung der Erfindung gehörend betrachtet werden. In den Zeichnungen zeigen:
Fig. 1a bis 1d:eine erste Variante eines Verfahrens nach der vorliegenden Erfindung mit einer ersten erfindungsgemässen Anordnung für einen ballon-expandierenden Stent,
Fig. 2a bis 2c: eine zweite Variante eines Verfahrens nach der vorliegenden Erfindung für einen selbst-expandierenden Stent,
Fig. 3: eine dritte Variante eines Verfahrens nach der vorliegenden Erfindung mit einer zweiten erfindungsgemässen Anordnung,
Fig. 4: eine Verpackung mit einer Umhüllung nach der vorliegenden Erfindung,
Fig. 5a, b, c: Abbildung einer histomorphometrischen Analyse eines Stents mit einheitlicher Reinheit gemäss der Erfindung,
Fig. 6a, b, c: Abbildung einer histomorphometrischen Analyse desselben Stenttyps, wie aus den Figuren 5a, b, c mit herkömmlicher Reinheit,
Fig. 7a: graphische Darstellung einer in-vivo Röntgenanalyse (Angiographie) eines ersten Stents gemäss der Erfindung,
Fig. 7b: graphische Darstellung einer in-vivo Röntgenanalyse (Angiographie) eines zweiten Stents gemäss der Erfindung und
Fig. 8a bis 8e: schematischer Ablauf des Einwachsens eines herkömmlichen blanken Metallstents (oben) und eines erfindungsgemässen hoch reinen blanken Metallstents (unten).

Nachfolgend wird die Bereitstellung eines Implantats zur Implantation nach der Erfindung am Beispiel eines Stents gezeigt. Im Allgemeinen ergibt sich im Fall eines Stents mit einem Ballonkatheter als Transporteinrichtung beispielsweise folgender Ablauf:
1. Stent wird in die schlauchförmige Umhüllung eingebracht.
2. Stent wird von einem definierten Medium umströmt
3. Ballonkatheter wird eingebracht, und wird ebenfalls umströmt
4. Nach einer Weile, wenn Stent und Ballon umströmt sind und sich somit keine Partikel mehr auf deren Oberflächen befinden, kann der Ballon in das Stentlumen hineingeschoben werden.
5. In einem Beispiel wird der Ballon etwas geöffnet (inflatiert), so dass der Stent durch Haftreibung auf dem Ballon verbleibt
6. Ballonkatheter mit Stent wird dann innerhalb der Umhüllung in eine Crimpeinheit geschoben und im Durchmesser reduziert - Stent und Ballon.
7. Ballonkatheter mit gekrimpetem oder montiertem Stent wird in die Verpackung geschoben, die Verpackung wird dicht verschlossen.

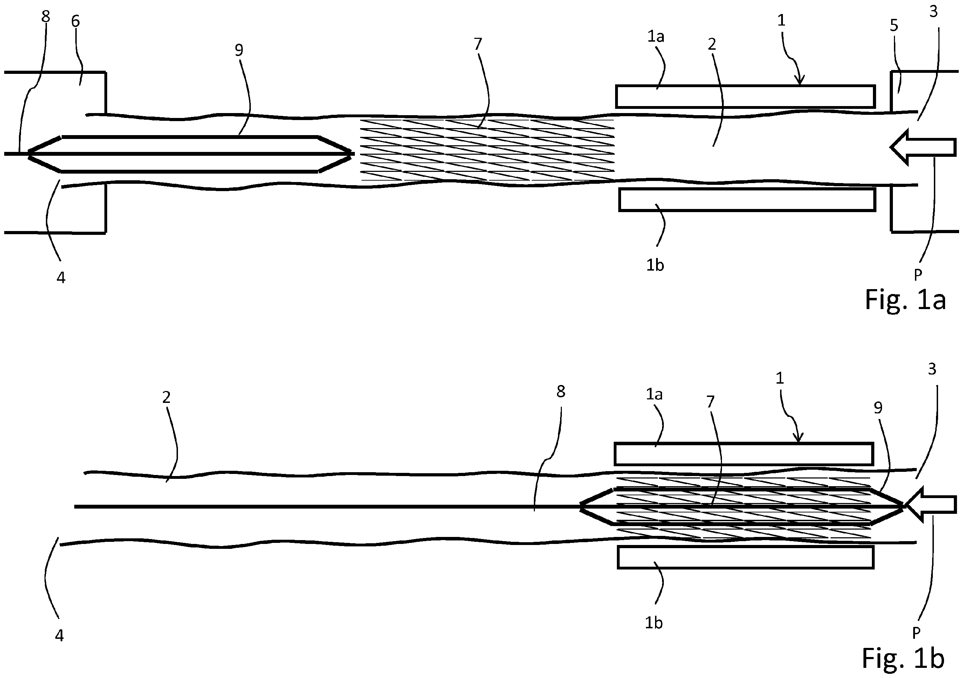

In den Figuren 1a bis 1d ist eine erste Variante zur Durchführung eines Verfahrens nach der vorliegenden Erfindung gezeigt, das zur Bereitstellung eines ballon-expandierenden Stents vorgesehen ist. Wie Figur 1a zu entnehmen ist, umfasst die Anordnung eine Bearbeitungseinheit in Form einer Crimpeinheit 1. Schematisch sind zwei Crimpelemente 1a und 1b der Crimpeinheit gezeigt, die Einheit weist selbstverständlich mehr als zwei Crimpelemente auf. Weiter umfasst die Anordnung eine Umhüllung 2, die sich durch die Crimpeinheit 1 und darüber hinaus entlang der Bearbeitungsachse der Crimpeinheit erstreckt und die eine vordere Öffnung 3 und eine hintere Öffnung 4 aufweist. Die Umhüllung 2 ist als länglicher Schlauch vorgesehen und ist biegsam sowie in ihrem Durchmesser verringerbar, bzw. kann gefaltet werden. An der vorderen Öffnung 3 ist eine Strömungseinheit 5 angeschlossen. Im Bereich der hinteren Öffnung 4 ist eine Reinigungseinheit 6 vorgesehen, wie z. B. Ultraschallelemente.

Die Strömungseinheit 5 erzeugt einen Strom eines definierten Mediums in Richtung des Pfeils P durch die Umhüllung. Innerhalb der Umhüllung 2 ist ein Implantat in Form eines Stents 7 und eine Transporteinrichtung in Form eines Dilatationskatheters 8 mit einem Dilatationsballon 9 angeordnet. Der Stent ist in einem expandierten Zustand, bevor er auf den Ballon komprimiert wird, gezeigt. Sowohl der Stent 7 als auch der Katheter 8 mit dem Ballon 9 haben die Reinigungseinheit durchlaufen, in der sie gereinigt wurden, und werden nun von dem Strom des definierten Mediums aus der Strömungseinheit 5 umströmt während sie in Transferrichtung in Richtung der Strömungseinheit innerhalb der Umhüllung transportiert werden. Stent und Transporteinrichtung werden somit entgegen der Stromrichtung innerhalb der Anordnung bewegt, was einer retrograden Bewegungsrichtung des Stromes in Bezug auf den Stent und die Transporteinrichtung entspricht. An der Strömungseinheit kann ein Filter nachgeschaltet werden, um auch Kontaminationspartikel aus der Strömungseinheit zurückzuhalten und eine Kontamination weiter zu verhindern.

Wie in Figur 1b gezeigt ist, wird in einem nächsten Verfahrensschritt der Ballon 9 des Katheters 8 innerhalb des Stents 7 angeordnet und beide in der Crimpeinheit 1 zwischen den Crimpelementen 1a und 1b gelagert. Während dessen umströmt das Medium weiterhin den Stent 7 und die Transporteinrichtung, so dass keine Verunreinigungen zwischen Stent und Ballon verbleiben und sich auf dem Stent ablagern können. Dabei können durch den retrograden Strom keine Kontaminationspartikel vom Schaft der Transporteinrichtung oder ähnlichem zwischen der Transporteinrichtung, z. B. dem Ballon, und dem Stent verbleiben. Der Strom erfolgt dabei entlang der Längsachse der Crimpeinheit 1 und tritt somit an einer Seite in diese ein und an einer gegenüberliegenden Seite wieder aus dieser aus. Eine Änderung der Bauteile der Crimpvorrichtung zur Durchführung der Umhüllung 2 und zur Durchleitung des Stroms ist daher nicht notwendig.

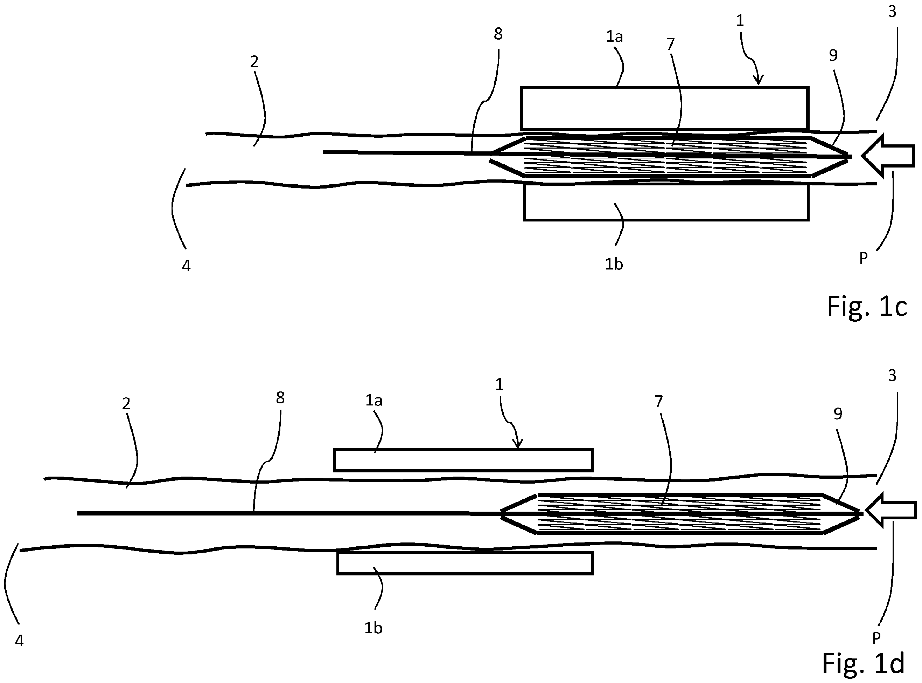

Im nächsten Verfahrenschritt gemäss Figur 1c komprimieren die Crimpelemente 1a und 1b der Crimpeinheit 1 den Stent 7, so dass dieser in seinem Durchmesser verringert wird und auf dem Ballon 9 angeordnet ist. Dabei verbleibt die Umhüllung 2 zwischen der äusseren Oberfläche des Stents 7 und den Crimpelementen der Crimpeinheit 1. Das definierte Medium umströmt weiterhin auch während des Komprimierens den Stent 7 bis dessen Oberflächen auf dem Ballon 9 aufliegen. Ein geringer Strom kann z. B. zwischen den Stegen des Stents hindurch strömen oder der Strom wird kurzzeitig bei einem Verschluss des Durchgangs der Umhüllung während des Komprimierens unterbrochen. Eine Ablagerung von Partikeln zwischen Ballon 9 und Stent 7 wird durch das vorbeiströmende Medium verhindert, welches Partikel mit sich trägt, die sich möglicherweise während des Crimpens z. B. vom Katheterschaft gelöst haben.

Als nächstes, wie in Figur 1d gezeigt, wird der auf den Ballon 9 komprimierte Stent 7 gemeinsam mit dem Katheter 8 aus der Crimpeinheit 1 innerhalb der Umhüllung 2 herausgeführt. Von dort kann er unmittelbar in eine Verpackung eingebracht werden. Die Umhüllung kann dabei zumindest teilweise in die Verpackung hinein ragen oder kann in eine Öffnung der Verpackung münden.

Der Stent wird in dieser Anordnung entgegen der Stromrichtung P bewegt, so dass der Stent und der vordere (distale) Teil der Transporteinrichtung immer mit frischem Medium umströmt werden.

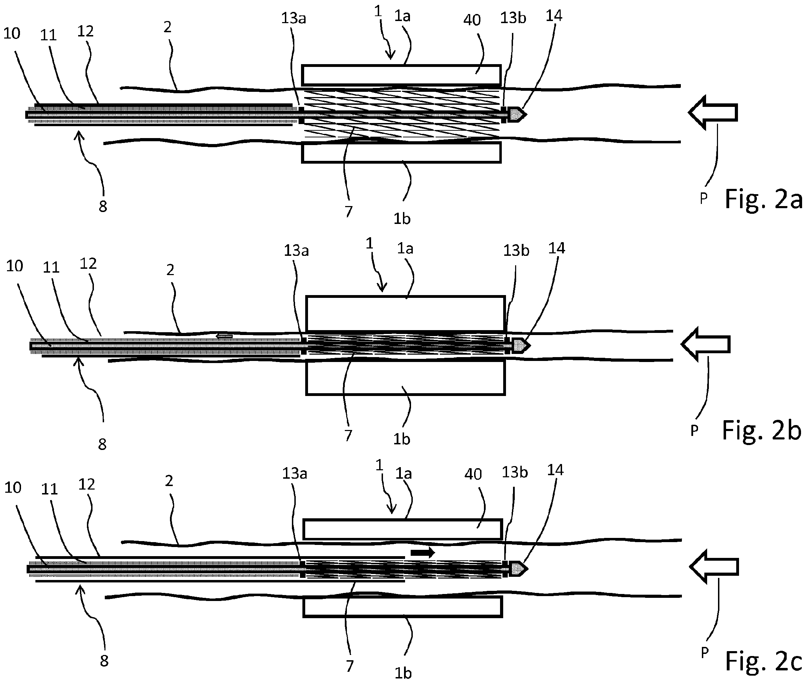

In den Figuren 2a bis 2c ist eine zweite Variante zur Durchführung eines Verfahrens nach der vorliegenden Erfindung gezeigt, das zur Bereitstellung eines selbst-expandierenden Stents vorgesehen ist. Die Anordnung zur Bereitstellung des Stents ist mit derjenigen der Figuren 1a bis 1d vergleichbar. Es ist wiederum eine Crimpeinheit 1 mit Crimpelementen 1a und 1b, eine Umhüllung 2, welche die Crimpeinheit 1 durchläuft, und ein Strom in Richtung des Pfeils P vorgesehen. Strömungseinheit und Reinigungseinheit sind zu Gunsten einer besseren Übersichtlichkeit nicht dargestellt. In der Umhüllung sind ein Stent 7 und eine Transporteinrichtung vorgesehen. Die Transporteinrichtung, bzw. der Schlauchkatheter, umfasst einen Innenschlauch 10, einen Stützschlauch 11 und einen Aussenschlauch 12. Auf dem Innenschlauch 10 ist ein proximaler Röntgenmarker 13a vorgesehen, welcher in herkömmlicher Weise zur Überprüfung der Position des Stents im Körperlumen dient. Am Ende des Innenschlauchs 10 ist eine Katheterspitze 14 angeordnet, welche den vordersten Punkt beim Einführen des Stents in ein Körperlumen definiert und ebenfalls einen (distalen) Röntgenmarker 13b besitzt. Der Innenschlauch 10 ist über einem Führungsdraht angeordnet und bildet einen Durchgang bis zur Spitze 14. Der Stützschlauch 11 schliesst sich an den proximalen Röntgenmarker 13a an. Der Aussenschlauch 12 ist über dem Stützschlauch 11 und dem Innenschlauch 10 angeordnet.

In Figur 2a sind der Stent und die Transporteinrichtung bereits gereinigt und in die Umhüllung 2 eingeführt. Der Stent 7 ist in einem expandierten Zustand über dem Innenschlauch 10 zwischen den beiden Röntgenmarkern 13a und 13b positioniert. Der Stentumfang ist in diesem Zustand noch grösser als der Innenumfang des Aussenschlauchs 12. Der Stent 7 und die Enden von Aussenschlauch 12 und Stützschlauch 11 sind vor den Crimpelementen 1a und 1b der Crimpeinheit gelagert.

Nun wird, wie in Figur 2b zu sehen, der Stent 7 mittels der Crimpeinheit 1 komprimiert. In komprimiertem Zustand liegt der Stent zwischen Marker 13a und 13b. Der Aussendurchmesser des komprimierten Stents 7 entspricht nun im Wesentlichen dem Durchmesser des Stützschlauchs 11. Der Stent wird von den Crimpelementen 1a und 1b in komprimiertem Zustand gehalten, während er unter seine Austenit-Formationstemperatur herabgekühlt wird, um den komprimierten Zustand zu halten. Zur Kühlung des Stents kann z. B. die Temperatur des definierten Mediums gekühlt werden oder es kann ein anderes Medium mit entsprechend tieferer Temperatur in die Umhüllung 2 eingeleitet werden, welches dem Strom des definierten Mediums beigemischt wird oder diesen ersetzt. Alternativ kann ausserhalb um die Umhüllung ein Kühlmedium vorgesehen werden.

Unterhalb der Austenit-Formationstemperatur bleibt der Stent in komprimiertem Zustand und die Crimpelemente 1a und 1b können geöffnet werden, ohne dass der Stent expandiert, wie in Figur 2c gezeigt ist. Der Durchmesser des Stents ist nun immer noch kleiner als der Innendurchmesser des Aussenschlauchs 12, so dass dieser über den Stent geschoben werden kann und somit ungewolltes Expandieren des Stents verhindert. Anschliessend kann der Stent in eine Verpackung transferiert werden.

Vor und während des Komprimierens des Stents und beim weiteren Transfer werden der Stent und auch die Transporteinrichung von dem Strom des definierten Mediums umströmt. Eine Kontamination des Stents und Ablagerungen zwischen Stent und Aussenschlauch und/oder Innenschlauch werden somit vermieden.

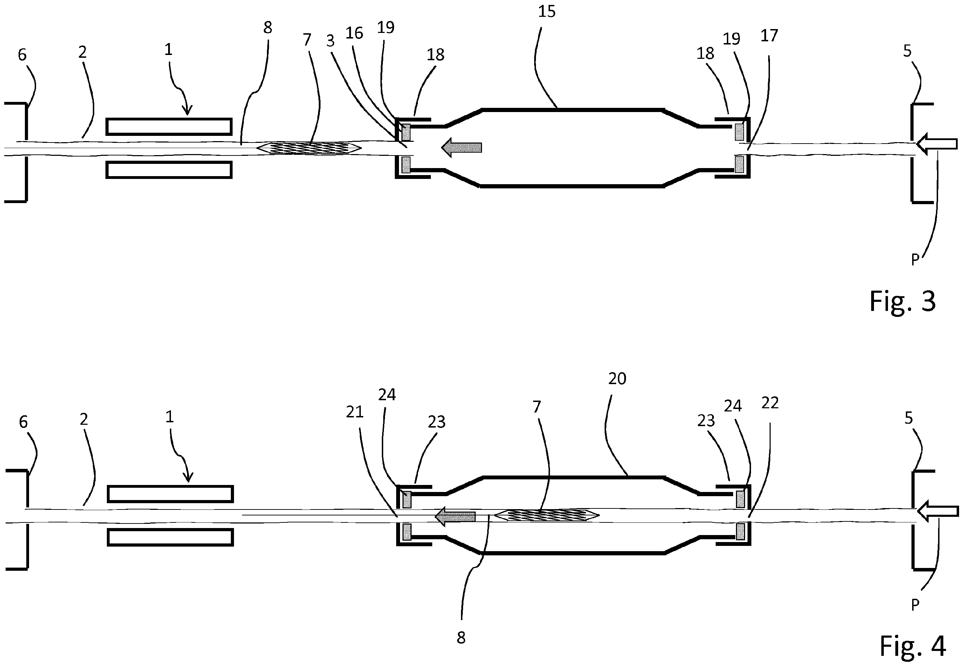

In Figur 3 ist eine weitere Anordnung zur Bereitstellung eines Stents zur Implantation nach der vorliegenden Erfindung gezeigt. Die Anordnung weist eine Crimpeinheit 1, eine Umhüllung 2, eine Strömungseinheit 5, eine Reinigungseinheit 6 und eine Verpackung 15 auf. Innerhalb der Umhüllung 2 sind eine Transporteinrichtung 8 und ein Stent 7 in komprimiertem Zustand gelagert und können innerhalb der Umhüllung 2 bewegt werden. Die Umhüllung 2 erstreckt sich von der, bzw. durch die Reinigungseinheit 6 und durch die Crimpeinheit 1 bis zu einer ersten Öffnung 16 der Verpackung 15 und ist an dieser Öffnung angeschlossen. An einer zweiten Öffnung 17, die der ersten Öffnung 16 gegenüberliegt, ist die Strömungseinheit 5 angeschlossen. Der Strom des definierten Mediums in Pfeilrichtung P strömt somit von der Strömungseinheit 5 durch die Verpackung 15 in die Umhüllung 2 und umströmt den Stent 7 und die Transporteinrichtung 8 innerhalb der Umhüllung 2. Der Stent 7 und die Transporteinrichtung 8 können innerhalb dieses Stromes in die Verpackung 15 eingeführt werden. Anschliessend werden die erste und die zweite Öffnung 16 und 17 der Verpackung verschlossen, so dass Stent und Transporteinrichtung innerhalb des schützenden Mediums in der Verpackung gelagert sind. Dabei ist es vorteilhaft zunächst die erste Öffnung 16 der Verpackung, an der die Umhüllung vorgesehen ist, zu schliessen, so dass durch die zweite Öffnung 17 frisches Medium in die Verpackung einströmen kann und in dieser verbleibt bis die zweite Öffnung 17 geschlossen und somit von der Strömungseinheit abgekoppelt wird. Zum Verschliessen der Öffnung 16 und 17 können Dichtungen 19, z.B. in Form von O-Ringen verwendet werden, welche durch den Verschluss (Deckel) 18 derart komprimiert werden, dass die Öffnung um die Transporteinheit auf beiden Seiten des Stents 7 abgedichtet wird. Der Deckel 18 kann z. B. durch einen Drehmechanismus mit einer Drehung relativ zum Verpackungsgehäuse die Dichtungen 19 komprimieren. Die Verpackung ist aus hartem Material gefertigt. So dient sie zum Schutz des Stents und der Transporteinrichtung vor mechanischen Einwirkungen und hält diese in ihrem Innenraum in einem definierten Medium, beispielsweise in einem teilweise flüssigen und teilweise gasförmigen Medium. Eine Kontamination des Stents bei der Vorbereitung zur Implantation wird so vermieden. Der Stent ist nun gemeinsam mit seiner Transporteinrichtung in der Verpackung zur Implantation bereit.

In Figur 4 ist eine alternative Verpackung 20 gezeigt, in der sich die Umhüllung 2 durch die Verpackung 20 erstreckt. Die Umhüllung verläuft durch eine erste Öffnung 21 und eine dieser gegenüber liegende zweite Öffnung 22 der Verpackung 20. Ein Strom in Richtung P umströmt den Stent 7 und die Transporteinrichtung 8 innerhalb der Umhüllung 2 und der Verpackung 20. An den Öffnungen 21 und 22 sind Verschlüsse 23 mit Dichtungen 24 vorgesehen, welche die Verpackung und die Umhüllung dicht verschliessen, beispielweise mit einem Drehmechanismus wie bei Figur 3 beschrieben. Die Verpackung 20 ist aus hartem Material gefertigt und dient als Schutzhülse für Stent und Transporteinrichtung. Die Umhüllung 2 umschliesst den Stent und die Transporteinrichtung und hält diese in einem definierten Medium.

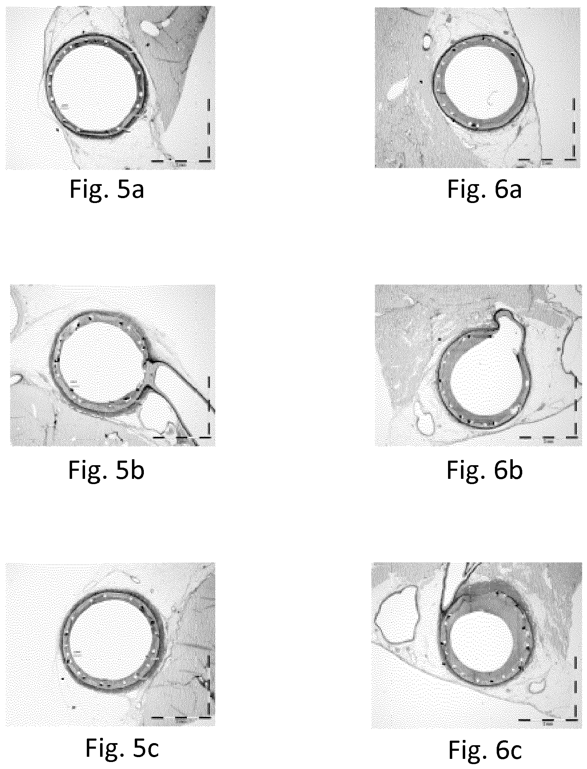

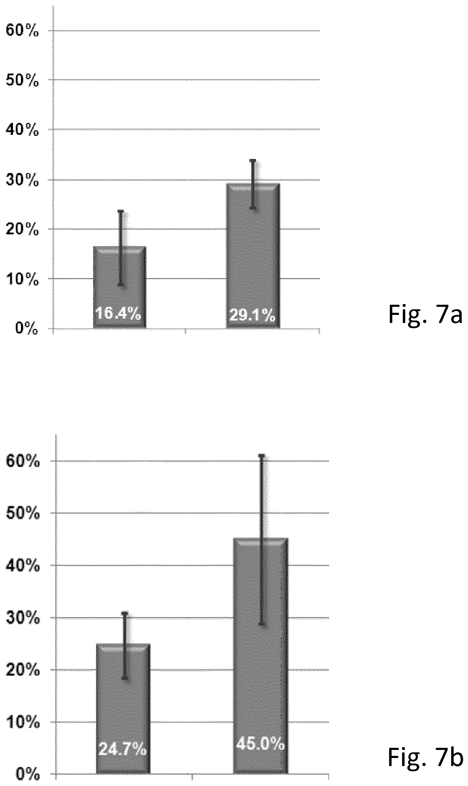

In den Figuren 5 bis 7 sind die Ergebnisse von Tierstudien gezeigt, die beim Einsetzen von Stents in Koronararterien im Schwein erhalten wurden. Bei Figur 7 wurde der geringste Gefässdurchmesser im Bereich des Stents unmittelbar nach dem Einsetzen eines Stents in eine Arterie und dreissig Tage nach dem Einsetzen und Einwachsen bestimmt. Nach dem Einsetzen des Stents verringert sich der Durchmesser des Stents auf Grund des Einwachsens von Zellen im Bereich des Stents. Der Gefässdurchmesser wird dabei angiographisch, d. h. mittels Röntgenanalyse erfasst. Die Studie umfasst eine Messreihe von acht Vergleichsversuchen.

In den Figuren 5a, 5b und 5c sowie 6a, 6b und 6c ist der Querschnitt eines koronaren Blutgefässes eines Schweines gezeigt, nachdem es 30 Tage nach dem Einsetzen eines Stents von Medtronic untersucht wurde, wie es für histologische Untersuchungen üblich ist. Dabei wird der ursprüngliche Gefässrand identifiziert und dann die Querschnittfläche der zugewachsenen Zellen berechnet, woraus sich eine prozentuale Zuwachsrate, d. h. die Endothelialisierung bzw. Stenoserate ergibt.

In den Figuren 5a, 5b und 5c ist ein Stent nach der vorliegenden Erfindung mit einer einheitlichen und hoch reinen Oberfläche verwendet worden, wie sich z. B. gemäss dem Verfahren der oben erwähnten parallelen Anmeldung verifizieren lässt. Es wurde ein kommerziell erhältlicher Stent des Herstellers Medtronic der Grösse 3x18mm verwendet und durch das erfindungsgemässe Verfahren bereitgestellt. Figur 5a zeigt den eingewachsenen Stent in einem proximalen Bereich des Blutflusses, Figur 5b in einem mittleren Bereich mit einer Bifurkation und Figur 5c in einem distalen Bereich des Blutflusses. Wie ersichtlich ist, besteht nur eine geringe Verkleinerung des Durchmessers der Arterie durch Zuwachsen. Zudem besteht ein gleichmässiges Einwachsen des Stents.

In den Figuren 6a, 6b und 6c ist ein herkömmlich bereitgestellter Stent verwendet worden, der keine einheitlich reine Oberfläche aufweist, wie ebenfalls durch das Verfahren der oben erwähnten parallelen Anmeldung verifiziert werden kann. Sowohl im proximalen Bereich (Figur 6a), im mittleren Bereich (Figur 6b) als auch im distalen Bereich (Figur 6c) wurde der Durchmesser deutlich mehr verengt als beim Stent aus den Figuren 5a, 5b und 5c. Insbesondere im distalen Bereich kann eine starke Restenose nachgewiesen werden.

Wie den Figuren zu entnehmen ist, lassen sich durch eine einheitliche und reine Stentoberfläche die Risiken bei der Behandlung von Gefässkrankheiten deutlich verringern. Zudem ist es mit dem Verfahren und der Vorrichtung nach der Erfindung möglich, die Oberflächencharakteristik von Stents gemäss einem Herstellungsverfahren oder nach einem bestimmten Präparationsprozess zuverlässig und ohne grossen Aufwand zu bestimmen. Somit können Stents, die keine ausreichend gute Oberflächencharakteristik zeigen, von einer Implantation ausgeschlossen werden.

In Figur 7a wurde ein Stent von Medtronic (3x18mm) mit einer spiralförmigen Gitterstruktur verwendet, der zum Implantieren in ein Körperlumen bereit war. Der linke Balken zeigt das Verhalten eines solchen Stents im Körperlumen nach einer Bereitstellung gemäss der Erfindung mit einem einheitlichen Oberflächenbenetzungsverhalten und der rechte Balken einen solchen Stent unmittelbar aus der Verpackung ohne weitere Behandlungsschritte, wie er herkömmlicher Weise verwendet wird. Es ist klar ersichtlich, dass der Stent gemäss der Erfindung mit einer einheitlichen Oberfläche ein verbessertes Einwachsverhalten zeigt, im Gegensatz zu einem herkömmlichen präparierten Stent. Eine unerwünschte Restenose kann in diesem Beispiel mit einem erfindungsgemässen Stent von 29.1% auf 16,4%, also um ca. 44% reduziert werden, wobei dies statistisch signifikant ist (p = 0,009).

In Figur 7b wurde ein Stent von Abbott der Grösse 3x18mm verwendet, der eine regelmässige symmetrische, also eine nicht helixartige, Gitterstruktur aufwies und zum Implantieren in ein Körperlumen bereit war. Der linke Balken zeigt wiederum das Verhalten eines solchen Stents im Körperlumen nach einer Bereitstellung gemäss der Erfindung und der rechte Balken einen solchen Stent unmittelbar aus der Verpackung ohne weitere Behandlungsschritte, wie herkömmlich verwendet. Auch der Stent mit dieser Gitterstruktur zeigt nach einer erfindungsgemässen Bereitstellung ein klar verbessertes Verhalten beim Einwachsen in die Arterie mit einer deutlich reduzierten Verengung des Durchmessers von 45% auf 24.7%, also um ca. 45%, wobei dies statistisch signifikant ist (p = 0,024).

Aus diesen Resultaten geht klar hervor, dass die Verwendung eines Stents nach der Erfindung mit einem einheitlichen, bzw. periodisch regelmässigen Oberflächenbenetzungsverhalten ein gegenüber herkömmlich zur Verfügung stehenden Stents deutlich verbessertes Einwachsverhalten und weniger Restenose zeigt. Nach der Erfindung wird daher vorgesehen blanke Metallstents mit einem einheitlichen, bzw. periodisch regelmässigen Oberflächenbenetzungsverhalten, also hochreine Stents für die Implantation in Körperlumina vorzusehen.

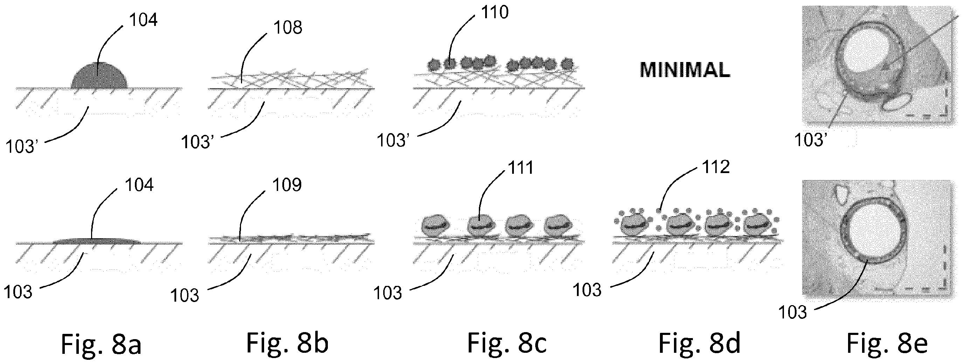

In den Figuren 8a bis 8e erfolgt eine schematische Darstellung der einzelnen Schritte beim Einwachsen eines Stents, wie sie bei Versuchen der Anmelderin identifiziert werden konnten. Es werden die einzelnen Schritte bei einem herkömmlichen blanken Metallstent 103' (oben) und einem hoch reinen blanken Metallstent 103, insbesondere bei einem Stent mit einem über die Gesamtlänge einheitlichen, bzw. periodisch regelmässigen Oberflächenbenetzungsverhalten gezeigt. Der hoch reine Stent wurde durch das Verfahren und die Anordnung gemäss der parallelen Anmeldung CH 00049/12 der Anmelderin erhalten. Im Beispiel gemäss den Figuren 8a bis 8e wird ein Stent mit einer hoch hydrophilen Oberfläche betrachtet.

In Figur 8a wird das Benetzungsverhalten eines Wassertropfens 107 auf der Stentoberfläche gezeigt. Auf dem herkömmlichen Stent 103' (Fig. 8a, oben) liegt ein grosser Kontaktwinkel vor während bei dem hoch reinen Stent 103 (Fig. 8a, unten) auf Grund der hydrophilen Oberflächeneigentschaften nur ein sehr geringer Kontaktwinkel entsteht. In Fig. 8b ist der Stent am Ort der Implantation platziert und die betrachtete Oberfläche ist gegenüber dem Blut exponiert. Beim herkömmlichen Stent 103' (Fig. 8b, oben) erfolgt zunächst eine Ablagerung von Proteinen, welche sowohl das Anhaften, als auch die Funktionalität von Neutrophilen verhindern (FGN, A2M, ApoA), so genannte Neutrophilinhibitoren 108. Beim hoch reinen Stent 103 (Fig. 8b, unten) sind die Neutrophilinhibitoren 108 (FGN, A2m und ApoA) stark reduziert und zugleich lagern sich sowohl Proteine ab, welche die Anhaftung von Thrombozyten verhindern (HMWK), als auch Proteine, welche die Anhaftung von Neutrophilen auf der Stentoberfläche fördern (PGN), so genannte Neutrophilpromotoren 109. Entsprechend werden anschliessend bei dem herkömmlichen Stent 103' (Fig. 7c, oben) auf den Neutrophilinhibitoren 108 hauptsächlich Thrombozyten 110 angesiedelt, welche grundsätzlich unerwünscht sind. Bei dem hoch reinen Stent 103 (Fig. 8c, unten) werden hingegen neutrophile Zellen 111 aus dem Blut des Patienten auf den Neutrophilpromotoren 109 angesiedelt, während Thrombozyten abgewiesen werden. Die aktivierten neutrophilen Zellen 111 sondern das Protein Cathelicidin (LL37) 112 auf der Stentoberfläche ab, vgl. Fig. 8d unten. Dadurch kann der Vorgang des Einwachsens positiv unterstützt werden, ohne dass hierfür eine Beschichtung oder eine Medikamentenabgabe erforderlich ist. Bei dem herkömmlichen Stent 103' wurde Cathelicidin nur in sehr geringem Mass gefunden. Die Untersuchungen haben gezeigt, dass der hoch reine Stent 103 zwei bis dreimal mehr Cathelicidin ansammelt als der herkömmliche Stent. Der hoch reine Stent 103 mit einem regelmässigen Oberflächenbenetzungsverhalten zeigt ein Einwachsverhalten, das vergleichbar mit dem aus den Figuren 5a bis 5c ist (siehe Fig. 8e, unten). Der herkömmliche Stent 103' jedoch zeigt ein Einwachsen gemäss der Figuren 6a bis 6c, also eine erneute Verengung des Durchgangs (siehe Fig. 8e, oben). Zusammenfassend lässt sich festhalten: die hoch reine Oberfläche des Stents 103 unterstützt und fördert diejenigen bioaktiven Prozesse, die zu einem gesunden und erwünschten Einwachsen des Stents 103 führen. Unerwünschte Prozesse werden dagegen eingedämmt oder unterbunden.

### Bezugszeichenliste

- 1: Crimpeinheit
- 2: Umhüllung
- 3: vordere Öffnung
- 4: hintere Öffnung
- 5: Strömungseinheit
- 6: Reinigungseinheit
- 7: Implantat, Stent
- 8: Transporteinrichtung
- 9: Ballon
- 10: Innenschlauch
- 11: Stützschlauch
- 12: Aussenschlauch
- 13a, 13b: Marker
- 14: Katheterspitze
- 15: Verpackung
- 16: erste Öffnung
- 17: zweite Öffnung
- 18: Verschluss
- 19: Dichtung
- 20: Verpackung
- 21: erste Öffnung
- 22: zweite Öffnung
- 23: Verschluss
- 24: Dichtung
- 103, 103': Stent
- 107: Wassertropfen
- 108: Neutrophilinhibitoren
- 109: Neutrophilpromotoren
- 110: Thrombozyten
- 111: Neutrophil-Zellen
- 112: Cathelicidin

- P: Stromrichtung

## Patentansprüche

1. Anordnung zur Bereitstellung eines Stents zur Implantation in ein Körperlumen umfassend:
- wenigstens eine Bearbeitungseinheit (1) zur Bearbeitung des Stents (7; 103),
- eine flexibel ausgebildete Umhüllung (2) mit einer vorderen Öffnung (3) und einer hinteren Öffnung (4), die zumindest teilweise an oder in der Bearbeitungseinheit (1) angeordnet ist, wobei der Stent (7; 103) innerhalb der Umhüllung (2) zwischen diesen Öffnungen (3, 4) gelagert werden kann,
- ein Katheter (8) zum Einführen des Stents in ein Körperlumen, der gemeinsam mit dem Stent (7; 103) innerhalb der Umhüllung (2) zwischen deren Öffnungen (3, 4) angeordnet ist, und
- wenigstens eine Strömungseinheit (5) zur Erzeugung eines Stroms (P) eines definierten Mediums durch die Umhüllung (2), die zumindest an eine Öffnung (3; 4) der Umhüllung (2) angeschlossen ist, wobei der Strom (P) den Stent (7; 103) innerhalb der Umhüllung (2) umströmt,
wobei die Bearbeitungseinheit (1) eine Einheit zum Komprimieren des Stents (7, 103) in Form einer Crimpeinheit (1) zum Aufbringen des Stents (7, 103) in oder auf den Katheter (8) umfasst.

2. Anordnung nach Anspruch 1, **dadurch gekennzeichnet, dass** die Umhüllung (2) schlauchartig geformt ist.

3. Anordnung nach einem Ansprüche 1 oder 2, **dadurch gekennzeichnet, dass** der Stent innerhalb der Umhüllung (2) beweglich gelagert ist und/oder die Umhüllung (2) innerhalb der Bearbeitungseinheit (1) beweglich ist und/oder die Umhüllung (2) aus der Bearbeitungseinheit entnehmbar ist.

4. Anordnung nach einem der vorhergehenden Ansprüche, **dadurch gekennzeichnet, dass** in oder an der Umhüllung (2) Haltemittel zur Positionierung des Stents (7; 103) innerhalb der Umhüllung vorgesehen sind.

5. Anordnung nach einem der vorhergehenden Ansprüche, **dadurch gekennzeichnet, dass** die Umhüllung (2) eine Wanddicke von 0.01mm bis 0.09mm, insbesondere von 0.02mm bis 0.06mm, aufweist.

6. Anordnung nach einem der vorhergehenden Ansprüche, **dadurch gekennzeichnet, dass** die Umhüllung (2) aus PTFE oder PFA besteht.

7. Anordnung nach einem der vorhergehenden Ansprüche, **dadurch gekennzeichnet, dass** sich die Umhüllung (2) durch die Bearbeitungseinheit (1), insbesondere eine Verpackungseinheit, erstreckt und an sich gegenüberliegenden Seiten der Bearbeitungseinheit (1) aus dieser austritt.

8. Anordnung nach einem der vorhergehenden Ansprüche, **dadurch gekennzeichnet, dass** mehrere Bearbeitungseinheiten (1; 6) in Folge vorgesehen sind und sich die Umhüllung durch die mehreren Bearbeitungseinheiten (1, 6) erstreckt.

9. Anordnung nach einem der vorhergehenden Ansprüche, **dadurch gekennzeichnet, dass** die Bearbeitungseinheit eine Reinigungseinheit (6) oder eine Verpackungseinheit bzw. eine Verpackung (15; 20) umfasst.

10. Anordnung nach einem der vorhergehenden Ansprüche, **dadurch gekennzeichnet, dass** eine Verpackungseinheit (15) in Strömungsrichtung zu vorderst nach einer Strömungseinheit (5) angeordnet ist und mit einer Öffnung (17) an die Strömungseinheit (5) und mit einer gegenüberliegenden Öffnung (16) an eine Öffnung der Umhüllung (2) angeschlossen ist.

11. Verfahren zur Bereitstellung eines Stents zur Implantation in ein Körperlumen, bei dem der Stent (7; 103) innerhalb einer Umhüllung (2) einer Bearbeitungseinheit (1), die eine Einheit zum Komprimieren des Stents (7, 103) in Form einer Crimpeinheit (1) zum Aufbringen des Stents in oder auf einen Katheter (8) umfasst, zugeführt wird, **dadurch gekennzeichnet, dass** der Stent (7; 103) zwischen einer vorderen Öffnung (3) und einer hinteren Öffnung (4) der flexibel ausgebildeten Umhüllung (2) gelagert und ein Strom (P) eines definierten Mediums durch die Umhüllung (2) geleitet wird, der der Stent (7; 103) innerhalb der Umhüllung (2) zumindest teilweise umströmt, und die Bearbeitungseinheit (1) von ausserhalb der Umhüllung (2) auf den Stent (7; 103) einwirkt.

12. Verfahren nach dem vorhergehenden Anspruch, **dadurch gekennzeichnet, dass** der Strom (P) den Stent (7; 103) umströmt während der Stent (7; 103) gereinigt, bearbeitet, einer Verpackung zugeführt und/oder zwischen Einheiten transferiert wird.

13. Verfahren nach einem der Ansprüche 11 oder 12, **dadurch gekennzeichnet, dass** der Stent (7; 103) während eines Transfers von einer Reinigungseinheit (6) zum Reinigen des Stents zu einer oder mehreren Bearbeitungseinheiten (1) und/oder zu einer Verpackung (15; 20) innerhalb der Umhüllung verbleibt.

14. Verfahren nach einem der Ansprüche 11 bis 13, **dadurch gekennzeichnet, dass** der Strom (P) des definierten Mediums retrograd in Bezug auf den Katheter des Stents (7; 103) innerhalb einer Anordnung zumindest bestehend aus einer Reinigungseinheit (6) sowie einer Bearbeitungseinheit (1) und/oder einer Verpackung (15; 20) erfolgt.

15. Verfahren nach einem der Ansprüche 11 bis 14, **dadurch gekennzeichnet, dass** die Temperatur des Stroms (P) während der Bearbeitung des Stents (7; 103) verändert wird.

16. Verfahren nach einem der Ansprüche 11 bis 15, **dadurch gekennzeichnet, dass** dem Strom (P) wenigstens eine zusätzliche Substanz beigefügt wird.

17. Verfahren nach einem der Ansprüche 11 bis 16, **dadurch gekennzeichnet, dass** eine Verpackungseinheit vorgesehen ist, in der der Stent (7; 103) innerhalb der Umhüllung (2) gemeinsam mit dieser in einer Verpackung (20) verpackt wird.

18. Verfahren nach einem der Ansprüche 11 bis 16, **dadurch gekennzeichnet, dass** eine Verpackungseinheit (15) in Strömungsrichtung zu vorderst nach einer Strömungseinheit (5) angeordnet ist und von dem definierten Medium durchströmt wird, wobei sich die Umhüllung (2) an die Verpackungseinheit (15) anschliesst.

## Claims

1. Arrangement for preparation of a stent for implantation in a body lumen comprising:
- at least a processing unit (1) for preparation of the stent (7; 103),
- a flexible sheath (2) with a front opening (3) and a rear opening (4), which is disposed at least partially on or in the processing unit (1), the stent (7; 103) being able to be stored inside the sheath (2) between these openings (3, 4),
- a catheter (8) for insertion of the stent (7; 103) into a body lumen, which catheter (8) is arranged together with the stent (7; 103) in the sheath (2) between its openings (3, 4), and
- at least one flow unit (5) for generation of a flow (P) of a defined medium through the sheath (2), which is connected at least at an opening (3; 4) of the sheath (2), the flow (P) flowing around the stent (7; 103) inside the sheath (2),
wherein the processing unit (1) comprises a unit for compression of the stent (7; 103) in the form of a crimp unit (1) for applying the stent (7; 103) in or on the catheter (8).

2. Arrangement according to claim 1, wherein the sheath (2) is designed tubular.

3. Arrangement according to claims 1 or 2, wherein the stent is movably arranged inside the sheath (2) and/or the sheath (2) is movable inside the processing unit and/or the sheath (2) is able to be removed from the processing unit (1).

4. Arrangement according to one of the preceding claims, wherein, in or on the sheath (2), holding means for positioning of the stent (7; 103) inside the sheath (2) are provided.

5. Arrangement according to one of the preceding claims, wherein the sheath (2) has a wall thickness of 0.01mm to 0.09 mm, in particular of 0.02 mm to 0.06 mm.

6. Arrangement according to one of the preceding claims, wherein the sheath (2) is composed of PTFE or PFA.

7. Arrangement according to one of the preceding claims, wherein the sheath (2) extending through the processing unit (1), in particular a packaging unit, and emerging therefrom at opposite sides of the processing unit (1).

8. Arrangement according to one of the preceding claims, wherein a plurality of processing units (1; 6) are provided in a series and the sheath (2) extends through the plurality of processing units (1, 6).

9. Arrangement according to one of the preceding claims, wherein the processing unit comprises a cleaning unit (6) or a packaging unit, or respectively a packaging (15; 20).

10. Arrangement according to one of the preceding claims, wherein a packaging unit (15) is disposed in flow direction at the front after a flow unit (5), and is connected by an opening (17) to the flow unit (5) and by an opposite opening (16) to an opening of the sheath (2).

11. Method for preparation of an stent for implantation in a body lumen, in which the stent (7; 103) is inserted inside a sheath (2) of a processing unit (1), which comprises a unit for compression of the stent (7, 103) in the form of a crimp unit (1) for applying the stent (7, 103) in or on the catheter (8), **characterized in that** the stent (7; 103) is stored between a front opening (3) and a rear opening (4) of the flexible sheath (2) and a flow (P) of a defined mediums is conducted through the sheath (2) which flows around the stent (7; 103) inside the sheath (2) at least partially, and the processing unit (1) acts upon the stent (7; 103) from outside the sheath (2).

12. Method according to the preceding claim, wherein the flow (P) flows around the stent (7; 103) while the stent (7; 103) is cleaned, prepared, fed to a packaging and/or transferred between units.

13. Method according to one of the claims 11 or 12, wherein the stent (7; 103) remains inside the sheath during a transfer from a cleaning unit (6), for cleaning of the stent, to one or more processing units (1) and/or to a packaging (15; 20).

14. Method according to one of the claims 11 to 13, wherein the flow (P) of the defined medium takes place in a retrograde way with respect to a transport direction of an stent (7; 103) within an arrangement consisting of at least a cleaning unit (6) as well as a processing unit (1) and/or a packaging (15; 20).

15. Method according to one of the claims 11 to 14, wherein the temperature of the flow (P) is changed during the preparation of the stent (7; 103).

16. Method according to one of the claims 11 to 15, wherein at least one additional substance is added to the flow (P).

17. Method according to one of the claims 11 to 16, wherein a packaging unit is provided, in which the stent (7; 103) inside the sheath (2) is packaged together therewith in a packaging (20).

18. Method according to one of the claims 11 to 16, wherein a packaging unit (15) is disposed in flow direction in the front after a flow unit (5) and the defined medium flows though it, the sheath (2) being connected to the packaging unit (15).

## Revendications

1. Agencement pour préparer un stent à implanter dans une lumière corporelle comprenant :
- au moins une unité de préparation (1) pour la préparation du stent (7 ; 103),
- une enveloppe (2) conçue de manière flexible munie d'une ouverture avant (3) et d'une ouverture arrière (4), qui est disposée au moins partiellement sur ou dans l'unité de préparation (1), le stent (7 ; 103) à l'intérieur de l'enveloppe (2) pouvant être placé entre ces ouvertures (3, 4),
- un cathéter (8) pour introduire le stent dans une lumière corporelle, qui est agencé conjointement avec le stent (7 ; 103) à l'intérieur de l'enveloppe (2) entre ses ouvertures (3, 4), et
- au moins une unité d'écoulement (5) pour produire un courant (P) d'un milieu défini, passant par l'enveloppe (2), qui est raccordée au moins à une ouverture (3 ; 4) de l'enveloppe (2), le courant (P) circulant autour du stent (7 ; 103) à l'intérieur de l'enveloppe (2),
l'unité de préparation (1) comprenant une unité pour comprimer le stent (7, 103) sous la forme d'une unité de sertissage (1) pour appliquer le stent (7, 103) dans ou sur le cathéter (8).

2. Agencement selon la revendication 1, **caractérisé en ce que** l'enveloppe (2) est conçue de manière tubulaire.

3. Agencement selon l'une des revendications 1 ou 2, **caractérisé en ce que** le stent est placé de manière mobile à l'intérieur de l'enveloppe (2) et/ou **en ce que** l'enveloppe (2) est mobile à l'intérieur de l'unité de préparation (1) et/ou **en ce que** l'enveloppe (2) peut être retirée de l'unité de préparation.

4. Agencement selon l'une quelconque des revendications précédentes, **caractérisé en ce que** des moyens de retenue pour positionner le stent (7 ; 103) à l'intérieur de l'enveloppe sont prévus dans ou sur l'enveloppe (2).

5. Agencement selon l'une quelconque des revendications précédentes, **caractérisé en ce que** l'enveloppe (2) présente une épaisseur de paroi de 0,01 mm à 0,09 mm, en particulier de 0,02 mm à 0,06 mm.

6. Agencement selon l'une quelconque des revendications précédentes, **caractérisé en ce que** l'enveloppe (2) est constituée de PTFE ou de PFA.

7. Agencement selon l'une quelconque des revendications précédentes, **caractérisé en ce que** l'enveloppe (2) s'étend dans l'unité de préparation (1), en particulier une unité d'emballage, et **en ce qu'**elle ressort de celle-ci au niveau des côtés opposés de l'unité de préparation (1).

8. Agencement selon l'une quelconque des revendications précédentes, **caractérisé en ce qu'**une pluralité d'unités de préparation (1 ; 6) sont prévues à la suite et **en ce que** l'enveloppe s'étend dans la pluralité d'unités de préparation (1, 6).

9. Agencement selon l'une quelconque des revendications précédentes, **caractérisé en ce que** l'unité de préparation comprend une unité de nettoyage (6) ou une unité d'emballage ou un emballage (15 ; 20).

10. Agencement selon l'une quelconque des revendications précédentes, **caractérisé en ce qu'**une unité d'emballage (15) est disposée dans le sens d'écoulement tout d'abord après une unité d'écoulement (5) et raccordée par une ouverture (17) à l'unité d'écoulement (5) et par une ouverture opposée (16) à une ouverture de l'enveloppe (2).

11. Procédé pour préparer un stent à implanter dans une lumière corporelle, dans laquelle le stent (7 ; 103) est inséré à l'intérieur d'une enveloppe (2) d'une unité de préparation (1) comprenant une unité pour comprimer le stent (7, 103) sous la forme d'une unité de sertissage (1) afin de placer le stent dans ou sur un cathéter (8), **caractérisé en ce que** le stent (7 ; 103) est placé entre une ouverture avant (3) et une ouverture arrière (4) de l'enveloppe (2) conçue de manière flexible, et un courant (P) d'un milieu défini est envoyé dans l'enveloppe (2) et circule au moins partiellement autour du stent (7 ; 103) à l'intérieur de l'enveloppe (2), et l'unité de préparation (1) agit sur le stent (7 ; 103) depuis l'extérieur de l'enveloppe (2).

12. Procédé selon la revendication précédente, **caractérisé en ce que** le courant (P) circule autour du stent (7 ; 103) pendant que le stent (7 ; 103) est nettoyé, préparé, introduit dans un emballage et/ou transféré entre des unités.

13. Procédé selon l'une des revendications 11 ou 12, **caractérisé en ce que** le stent (7 ; 103) reste à l'intérieur de l'enveloppe pendant un transfert depuis une unité de nettoyage (6) destinée à nettoyer le stent vers une ou plusieurs unités de préparation (1) et/ou vers un emballage (15 ; 20).

14. Procédé selon l'une des revendications 11 à 13, **caractérisé en ce que** le courant (P) du milieu défini se déplace de manière rétrograde par rapport au cathéter du stent (7 ; 103) à l'intérieur d'un agencement au moins constitué d'une unité de nettoyage (6) ainsi que d'une unité de préparation (1) et/ou d'un emballage (15 ; 20).

15. Procédé selon l'une des revendications 11 à 14, **caractérisé en ce que** la température du courant (P) est modifiée durant la préparation du stent (7 ; 103).

16. Procédé selon l'une des revendications 11 à 15, **caractérisé en ce qu'**on ajoute au moins une substance additionnelle au courant (P).

17. Procédé selon l'une des revendications 11 à 16, **caractérisé en ce qu'**une unité d'emballage est prévue, dans laquelle le stent (7 ; 103) à l'intérieur de l'enveloppe (2) est emballé conjointement avec celle-ci dans un emballage (20).

18. Procédé selon l'une des revendications 11 à 16, **caractérisé en ce qu'**une unité d'emballage (15) est agencée dans le sens d'écoulement tout d'abord après une unité d'écoulement (5) et traversée par le milieu défini, l'enveloppe (2) étant raccordée à l'unité d'emballage (15).
